(19) Europäisches Patentamt
European Patent Office
Office européen des brevets

(11) **EP 2 717 756 B1**

(12) **EUROPÄISCHE PATENTSCHRIFT**

(45) Veröffentlichungstag und Bekanntmachung des Hinweises auf die Patenterteilung:
**06.02.2019 Patentblatt 2019/06**

(21) Anmeldenummer: **12729412.2**

(22) Anmeldetag: **07.06.2012**

(51) Int Cl.:
*A61B 5/00* *(2006.01)*      *A61B 5/0205* *(2006.01)*
*A61B 5/022* *(2006.01)*      *A61B 5/024* *(2006.01)*
*A61B 5/0408* *(2006.01)*      *A61B 5/1455* *(2006.01)*

(86) Internationale Anmeldenummer:
**PCT/EP2012/060836**

(87) Internationale Veröffentlichungsnummer:
**WO 2012/168388 (13.12.2012 Gazette 2012/50)**

(54) **SENSOR ZUR MESSUNG VON VITALPARAMETERN IM GEHÖRGANG**

SENSOR FOR MEASURING VITAL PARMETERS IN THE AUDITORY CANAL

CAPTEUR POUR MESURER DES PARAMÈTRES VITAUX DANS LE CONDUIT AUDITIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.06.2011 DE 102011077066**
**30.08.2011 DE 102011081815**

(43) Veröffentlichungstag der Anmeldung:
**16.04.2014 Patentblatt 2014/16**

(73) Patentinhaber: **Cosinuss GmbH**
**81379 München (DE)**

(72) Erfinder: **KREUZER, Johannes**
**81379 München (DE)**

(74) Vertreter: **Franke, Dirk**
**Franke & Partner**
**Patent- und Rechtsanwälte**
**Widenmayerstraße 25**
**80538 München (DE)**

(56) Entgegenhaltungen:
**EP-A2- 1 922 989      DE-A1-102007 046 295**
**US-A1- 2009 221 888      US-A1- 2010 191 144**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft einen Sensor zur Messung eines Vitalparameters im Gehörgang eines Menschen oder eines Tieres. Zudem betrifft die Erfindung ein Verfahren zur Bestimmung gewebsoptischer Größen, vorzugsweise der Sauerstoffsättigung im Blut, insbesondere zur Durchführung von Pulsoximetrie, vorzugsweise Transmissions-Pulsoximetrie, im Gehörgang.

Eine Messung wichtiger physiologischer Parameter bei Mensch oder Tier, insbesondere kontinuierlich und unter mobilen Bedingungen, ist in der modernen Medizin nicht mehr wegzudenken. Dazu zählen insbesondere die Messung der Körperkemtemperatur oder Körpertemperatur, gewebsoptischer Parameter, wie die arterielle Sauerstoffsättigung, die Herzfrequenz und die Atemfrequenz, die Konzentration bestimmter Stoffe im Blut und/oder Gewebe, aber auch die Messung mechanischer Parameter, wie Lage, Position und Beschleunigung. Als Messort für derartige nicht-invasive Messungen hat sich der Gehörgang als geeignet erwiesen.

Viele diagnostische und therapeutische Maßnahmen, viele operative Eingriffe, die früher stationär durchgeführt wurden, werden heutzutage zumeist ambulant ausgeführt oder doch wenigstens mit deutlich kürzeren Hospitalisierungszeiten. Es besteht der Bedarf, den ambulanten Patienten mehr oder weniger kontinuierlich zu überwachen, um seine Gesundheit sicherzustellen und zugleich eine Gefährdung außerhalb des Krankenhauses zu erkennen. Viele Gefährdungen begleiten den Alltag, wie beispielsweise Diabetes, Allergien, Hypertonie oder Fettstoffwechselstörungen.

Die Messung der Körpertemperatur wird zumeist nur punktuell und diskontinuierlich durchgeführt. Prinzipiell kann die Messung der Körpertemperatur an verschiedenen mehr oder weniger geeigneten Orten erfolgen, wobei dann die gemessene Temperatur mehr oder weniger relevant für das ist, was als "Körper(kem)temperatur" bezeichnet wird. Unter Körper(kem)temperatur wird einerseits eine im räumlich-anatomischen Sinne zentrale Temperatur, beispielsweise eine Temperatur der innersten Organe, verstanden, die nicht durch unmittelbare äußere Einflüsse gestört ist. Andererseits versteht man darunter auch eine nahe dem Hypothalamus gemessene Temperatur, weil hier der physiologische Sensor für die Temperaturregelung des Menschen sitzt und sie somit für den thermischen Haushalt des Organismus die größte Bedeutung hat.

Die im Stand der Technik beschriebenen Strahlungssensoren sind nicht sehr genau und zumeist werden Messungen der Körpertemperatur am Ohr nur punktuell durchgeführt, d.h. eine diskontinuierliche Messung erfolgt. Die Körpertemperatur ist eine wichtige physiologische Größe zur allgemeinen Überwachung, da sie viele wichtige Zustände des Körpers widerspiegelt.

Die Pulsoximetrie ist ein optisches Verfahren zur Bestimmung der Sauerstoffsättigung über die Photometrie des Gewebes und ist weit verbreitet und bereits miniaturisiert. Eine mobile Pulsoximetrie scheitert zumeist am Messort, denn der Messort ist fast immer der Finger. Andere Messorte sind weniger zuverlässig. Es besteht ein hoher Bedarf eine mobile, im Alltag nicht störende Pulsoximetrie zur Verfügung zu stellen. Die aus dem Stand der Technik bekannten Vorrichtungen stellen meist ein ungünstiges und komplexes Design dar, welche erhebliche Fehlerquellen umfassen. Zumeist versprechen solche Vorrichtungen wenig Halt. Dazu kommt, dass der Sensor zumeist mechanisch instabil ist und die mechanische Beziehung zur Ohrmuschel fehlt. Strahlungssensoren, die im Gehörgang positionierbar sind und mit denen eine eine kontinuierliche Messung des Vitalparameters im Gehörgang erfolgt, sind beispielsweise aus den Dokumenten EP-A-1,922,989 oder DE 10 2007 046295 bekannt.

Es ist die Aufgabe der Erfindung, einen Sensor bereitzustellen, der dazu geeignet ist, mobile Personen zu überwachen, die ihren Alltag absolvieren, wobei der Sensor zugleich kosmetisch unauffällig sein soll und kontinuierlich oder häufig Vitalparameter misst. Außerdem soll eine Möglichkeit bereitgestellt werden, gewebsoptische Größen im Gehörgang zu bestimmen. Hierbei bedeutet der Ausdruck "kontinuierlich", dass die Häufigkeit von Messungen derart hoch ist, dass Änderungen der Messgröße mit einer Frequenz abgetastet werden, die für alle diagnostischen und/oder therapeutischen Zwecke ausreichend ist, so dass eine quasi-kontinuierliche Aussage möglich ist. Zugleich soll der mobile Sensor im Stromverbrauch so bemessen sein, dass akzeptable Betriebszeiten mit verfügbaren Energiezellen verfügbar werden.

Diese Aufgabe wird dadurch gelöst, dass ein Sensor zur Messung eines Vitalparameters im Gehörgang eines Menschen oder eines Tieres bereitgestellt wird, wobei der Sensor umfasst: einen Sensorelementaufsatz, der wenigstens teilweise im Gehörgang positionierbar ist, ein Sensorelement, das verbindbar mit dem Sensorelementaufsatz ist und wenigstens teilweise im Gehörgang positionierbar ist, und ein Positionierungselement, geeignet zum Positionieren des Sensorelementaufsatzes in dem Gehörgang, wobei das Positionierungselement an wenigstens einem Ende mit dem Sensorelementaufsatz verbindbar ist, wobei die Position des Sensorelements im Gehörgang durch die Eindringtiefe des Sensorelementaufsatzes in dem Gehörgang bestimmt wird, und wobei das Positionierungselement eine geeignete, die Anatomie der Ohrmuschelinnenseite nachvollziehende Formgebung aufweist, und zum Drücken des Sensorelementaufsatzes in das Innere des Gehörgangs geeignet ist, derart, dass der Sensor stabil gehalten wird und eine kontinuierliche Messung des Vitalparameters im Gehörgang erfolgt.

Vorzugsweise wird der äußere Gehörgang als Messort herangezogen, der sich durch die Kombination von physiologischen Eigenschaften einerseits mit mechanischen, technischen und/oder funktionellen Merkmalen andererseits gerade für die mobile bzw. die kontinuierliche Sensorik eignet. Der Ausdruck, dass ein Vitalparameter gemessen wird, bedeutet, dass wenigstens ein, d.h. auch zwei oder mehrere Parameter gleichzeitig gemessen werden können. Der Ausdruck, dass das Sensorelement verbindbar mit dem Sensorelementaufsatz ist, bedeutet, dass das Sensorelement mit dem Sensorelementaufsatz, insbesondere über weitere Komponenten des Sensors oder auch über Leitungen, Drähte, Kabel oder dergleichen, verbunden werden kann. Das Sensorelement kann aber auch in direktem Kontakt mit dem Sensorelementaufsatz stehen, d.h. direkt an dem Sensorelementaufsatz angebracht sein. Vorzugsweise umfasst das Sensorelement den Sensorelementaufsatz, d.h. der Sensorelementaufsatz ist als ein Teil des Sensorelements ausgestaltet. Der Ausdruck, dass die Position des Sensorelements im Gehörgang durch die Eindringtiefe des Sensorelementaufsatzes in dem Gehörgang bestimmt wird, bedeutet, dass die Positionierung des Sensorelements relativ zum Sensorelementaufsatz erfolgt, so dass eine genaue und kontinuierliche Messung des Vitalparameters erfolgen kann, insbesondere sind Sensorelement und Sensorelementaufsatz nahe zueinander positioniert, so dass eine Signalübertragung zwischen Sensorelementaufsatz und Sensorelement einwandfrei funktionieren kann. Vorzugsweise sind Positionierungselement und Sensorelementaufsatz einstückig ausgebildet.

Unter dem Ausdruck "Sensorelement" wird der Sensor, also der eigentliche Messfühler, verstanden, der die eigentliche physiologische bzw. biologische Größe in ein elektrisches Signal umwandelt, beispielsweise ein Wandler, ein Halbleiter, ein Messfühler oder Elektroden. Unter dem Ausdruck "Sensorelementaufsatz" und "Positionierungselement" werden funktionell und/oder mechanisch wirkverbundene Vorrichtungen verstanden, die zusammen das Sensorelement relativ zur Gehörgangswand derart positionieren, dass der physiologische bzw. biologische Parameter möglichst störungsarm gemessen werden kann. Aufgabe des Sensorelementaufsatzes ist es, die radiale Position des Sensorelements zu definieren, während es Aufgabe des Positionierungselements ist, die axiale Position zu definieren. Gemäß anderen bevorzugten Ausführungsbeispielen der Erfindung sind beide Funktionen nicht trennbar bzw. sie verschmelzen miteinander. Der Ausdruck "Sensor" steht insbesondere für die Kombination aus Sensorelement, Sensorelementaufsatz und Positionierungselement. Gemäß anderen bevorzugten Ausführungsbeispielen der Erfindung ist mit dem Ausdruck "Sensor" auch die Auswerteeinheit gemeint, die vorzugsweise hinter dem Ohr sitzt und dazu geeignet ist, dem Sensor Halt zu geben.

Gemäß einem bevorzugten Ausführungsbeispiel der Erfindung umfasst der Sensor ein Stabilisierungselement, beispielsweise einen Haltefaden, das zum Halt des Sensors in der Ohrmuschel geeignet ist und zumindest teilweise an dem Positionierungselement und/oder an dem Sensorelementaufsatz befestigbar ist. Vorzugsweise ist das Stabilisierungselement wenigstens teilweise mit einer Feder verbindbar, wobei die Feder dazu geeignet ist, das Stabilisierungselement gegen die Ohrmuschel zu drücken.

Gemäß einem anderen bevorzugten Ausführungsbeispiel der Erfindung ist der Sensorelementaufsatz derart elastisch ausgestaltet, dass er über seine Federkraft einen definierten Andruck auf die Wand des Gehörgangs ausübt, so dass sich seine Form der Form des Gehörgangs anpasst.

Erfindungsgemäß umfasst der Sensor eine Kontrolleinheit zur Überprüfung der Positionierung des Sensors im Gehörgang, wobei anhand der zeitlichen Änderung des Messsignals bei kontinuierlicher Messung des Vitalparameters die Position des Sensorelements und/oder des Sensorelementaufsatzes bestimmt wird und nach einer Änderung des Messsignals um einen vorbestimmten Wert die Abweichung der aktuellen Position von der gewünschten Position über eine Anzeigeeinheit signalisiert wird.

Zudem umfasst der Sensor eine Kontrolleinheit zur Überprüfung der Positionierung des Sensors im Gehörgang, wobei die Kontrolleinheit die optimale Position des Sensors bestimmt und dies über die Anzeigeeinheit signalisieren kann. Vorzugsweise ist eine Auswerteeinheit vorgesehen, die an einem von dem Sensorelement abgewandten Ende des Positionierungselements an der Ohrmuschel positionierbar ist und geeignet ist, den Sensor stabil zu halten. Vorzugsweise versendet die Auswerteeinheit nach Digitalisierung des analogen Messsignalverlaufs, die daraus gewonnenen Informationen an weitere Peripherieeinheiten, insbesondere an eine Mobilfunkeinheit und/oder einen Computer. Vorzugsweise umfasst der Sensor einen Lautsprecher, der geeignet ist, ein Biofeedback über den gemessenen Vitalparameter im Gehörgang wieder an das Innere des Gehörgangs zurückzugeben. Der gemessene Vitalparameter, beispielsweise die Pulsfrequenz, dient vorteilhafterweise dazu, der Person zu signalisieren, ob er oder sie z.B. beim Joggen schneller oder langsamer laufen soll, je nachdem ob der gemessene Puls zu hoch oder zu niedrig ist. Musik oder andere akustische Wellen werden vorzugsweise zeitgleich übertragen, insbesondere im Bereich zwischen 0 und 20 kHz.

Gemäß einem anderen bevorzugten Ausführungsbeispiel der Erfindung sind der Sensorelementaufsatz und das Sensorelement in Form eines Lichtmitters und eines Lichtempfängers ausgestaltet, wobei der Sensor weiter Durchstrahlungsmittel aufweist, das geeignet ist, zu verhindern, dass Licht direkt vom Lichtemitter ohne Durchstrahlung des Gewebes zum Lichtempfänger gelangt. Wenn Licht vom Lichtemitter zum Lichtempfänger auf direktem Wege zum Lichtempfänger gelangt, spricht der Fachmann von "Shuntlicht". "Shuntlicht" bedeutet, dass Licht vom Lichtemitter zum Lichtempfänger auf direktem Weg zum Lichtempfänger gelangt, ohne auf seinem Weg durchblutetes Gewebe durchstrahlt zu haben. Bei einer Transmissions-Pulsoximetrie kommt üblicherweise kein Shuntlicht vor, weil kein Licht den Empfänger erreicht, das nicht-durchblutetes Gewebe durchstrahlt hat. Vorzugsweise umfasst das Durchstrahlungsmittel ein scheibenförmiges oder ein schirmförmiges Element, an dem der Lichtemitter an einer Seite und der Lichtempfänger an einer anderen Seite angeordnet ist und/oder das Durchstrahlungsmittel umfasst einen schirmförmigen Doppelsensorelementaufsatz, bei dem der Lichtemitter an dem ersten Schirm und der Lichtempfänger an dem zweiten Schirm vorgesehen sind. Vorzugsweise ist der Sensorelementaufsatz weiter dazu geeignet, eine Maximierung der Lichtweglänge bzw. eine Unterdrückung verkürzter Lichtwege zur lichtmindernden, besonders vorzugsweise lichtundurchlässigen, Abdeckung von mehr als der Hälfte des halben inneren Gehörgangsumfangs im Bereich des Nettolichtwegs zu beiden Seiten des Lichtempfängers zu bewirken.

Dabei versteht der Fachmann unter "Netto-Lichtweg" den kürzesten Lichtweg, den das Licht vom Austritt aus dem Lichtemitter bis zum Eintritt in den Lichtempfänger benutzen kann, wobei Streu- und Beugungsphänomene im vitalen Gewebe berücksichtigt werden. Auf dem Weg vom Lichtemitter zum Lichtempfänger sind beliebig viele Ausbreitungswege unterschiedlicher Länge abhängig von den multiplen Streuzentren möglich, an denen Lichtstrahlen auf diesem Weg gebeugt, gebrochen und/oder gestreut werden. Der "Netto-Lichtweg" repräsentiert den Lichtweg mit der höchsten Lichtintensität am Lichtempfänger, wobei Streu- und Beugungsphänomene berücksichtigt werden. Erfindungsgemäß ist der Sensor größenverstellbar ausgestaltet, wobei der Sensorelementaufsatz und das Positionierungselement jeweils austauschbar und/oder größenverstellbar ausgestaltet sind. Dies trägt zur leichten Austauschbarkeit der einzelnen Komponenten und/oder Anpassung an die individuellen anatomischen Gegebenheiten der Person oder des Tieres bei, insbesondere auch aus hygienischen Gründen.

Vorzugsweise ist der Sensorelementaufsatz gewölbt und/oder schirmförmig ausgestaltet, besonders vorzugsweise umfasst der Sensorelementaufsatz als Material Silikon.

Vorzugsweise umfasst der Vitalparameter mindestens einen physiologischen, biochemischen und/oder bioelektrischen Parameter, insbesondere ist der Vitalparameter aus der Gruppe ausgewählt umfassend die Körpertemperatur, die Sauerstoffsättigung des Blutes, die Herzfrequenz, einen herzelektrischen Parameter, die Atemfrequenz, die Konzentration von im Blut gelöster Substanzen, insbesondere der arteriellen Sauerstoffsättigung, die Konzentration von im Gewebe vorhandenen Substanzen, die körperliche Aktivität und die Körperlage.

Gemäß einem anderen Aspekt der Erfindung wird die Aufgabe dadurch gelöst, dass ein Verfahren zur Bestimmung gewebsoptischer Größen, vorzugsweise der Sauerstoffsättigung im Blut, insbesondere zur Durchführung von Pulsoximetrie, vorzugsweise Transmissions-Pulsoximetrie, im Gehörgang bereitgestellt wird, wobei das Verfahren den Schritt umfasst: die Optimierung der Hilfsgröße $\Omega$ zur Bestimmung der Sauerstoffsättigung, vorzugsweise auf einen Wert von $\leq 0{,}5$, besonders vorzugsweise auf einen Wert $\geq 0{,}4$ und $\leq 0{,}5$, ganz besonders vorzugsweise auf einen Wert von ungefähr 0,45, für humanes Oxihämoglobin und/oder für eine Wellenlänge, vorzugsweise von 740 nm und/oder 880 nm, oder für symmetrische Spektren von lichtemittierenden Dioden gleicher Zentralwellenlängen.

Der Bereich $\geq 0{,}4$ und $\leq 0{,}5$ ergibt vorteilhafterweise, dass hier rechnerisch und messtechnisch die gleichen Werte entstehen. Dadurch werden insbesondere Messfehler verringert und die Messung somit genauer.

Das Verfahren wird vorzugsweise unter Einspeisung von akustischen Wellen, insbesondere im Bereich zwischen 0 und 20 kHz, ausgeführt.

Vorzugsweise werden mindestens zwei Wellenlängen zwischen 660 nm und 1000 nm gewählt, besonders vorzugsweise Wellenlängen um 740 nm und/oder um 880 nm, ganz besonders vorzugsweise eine Wellenlänge $\leq 810$ nm und eine Wellenlänge > 810 nm. Vorteilhafterweise wird die Wellenlänge oder der Wellenlängenbereich so gewählt, dass insbesondere dort ein großer Abstand zwischen den Absorptionskoeffizienten besteht und/oder die spektrale Absorptionskurve in diesem Bereich flach ist. Vorteilhafterweise wird dadurch auch die Störanfälligkeit reduziert und der Messbereich aufgespreizt.

Die Modulationstiefe MD für das monochromatische Licht bzw. für das Lichtspektrum entsteht durch Division von Wechsellichtspektrum, auch als AC bezeichnet, durch das Gleichlichtspektrum, auch als DC bezeichnet:

$$\mathrm{MD}_{\lambda} = \frac{\mathrm{AC}_{\lambda}}{\mathrm{DC}_{\lambda}}.$$

Vorzugsweise lassen sich mit der Pulsoximetrie unter anderem die Pulsfrequenz und/oder die Atemfrequenz bestimmen. Aber auch andere gewebsoptische Parameter lassen sich bestimmen. Werden zwei oder mehr Spektralbereiche verwendet und werden die jeweiligen Modulationstiefen bei entsprechenden Wellenlängen zueinander in Beziehung gesetzt, so ergibt sich aus den jeweiligen Modulationstiefen eine Variable $\Omega$, im Englischen auch als Ratio bezeichnet:

$$\Omega = \frac{\mathrm{MD}_{\lambda_1}}{\mathrm{MD}_{\lambda_2}} \quad \text{und} \quad \Omega = \frac{\left(\frac{\mathrm{AC}}{\mathrm{DC}}\right)_{\lambda_1}}{\left(\frac{\mathrm{AC}}{\mathrm{DC}}\right)_{\lambda_2}}.$$

Mit diesen Werten lässt sich vorzugsweise die arterielle Sauerstoffsättigung, die venöse Sauerstoffsättigung und/oder die arteriell-venöse Sättigungsdifferenz ermitteln.

Die Wahl des äußeren Gehörgangs als Messort ist in vielfacher Hinsicht günstig, beispielsweise sind Tragekomfort und Trage-Kosmetik im äußeren Gehörgang sehr günstig und Bewegungen des Kopfes im Vergleich zu Extremitäten sind eher gering und niederfrequent. Außerdem ist der äußere Gehörgang gut durchblutet, was für optischplethysmographische Messprinzipien, wie die Pulsoximetrie, geeignet ist. Vorzugsweise wird der Schall weitgehend ungehindert durch die verschiedenen Komponenten der Sensorik ans Trommelfell gelangen. Dies geschieht vorzugsweise dadurch, dass die verschiedenen Komponenten der Sensorik den Schall kaum oder nicht nennenswert dämpfen, und/oder dass der Sensorelementaufsatz so konstruiert ist, dass er die Schallfortleitung in Richtung auf das Trommelfell entweder zulässt, d.h. nicht wahrnehmbar behindert, oder sie sogar aktiv herstellt. Vorzugsweise ist der Sensorelementaufsatz mit geeigneten Löchern und/oder Aussparungen und/oder Kanälen vorgesehen, die gewährleisten, dass der äußere Gehörgang nicht vollständig verschlossen oder gedämpft wird.

Vorzugsweise definiert das Positionierungselement die axiale Position des Sensorelements im Gehörgang, d.h. ihre Tiefe im Gehörgang, was eine für das Messergebnis geeignete Größe darstellt. Vorteilhafterweise dient das Positionierungselement zum Halt des Sensors, d.h. ist für die mechanische Stabilität des Sensors zuständig. Vorzugsweise findet die Auswerteeinheit hinter dem Ohr Halt durch Einklemmung über geeignete Klemmmittel gegen den Kopf. Alle Komponenten sind vorzugsweise hautfarben eingefärbt oder durchsichtig, so dass der Sensor besonders unauffällig ist. Die Auswerteeinheit umfasst vorzugsweise die Energiezellen sowie einen Teil der Elektronik für die Signalverarbeitung.

Nachfolgend wird die Erfindung anhand bevorzugter Ausführungsbeispiele unter Bezugnahme auf die Zeichnungen weiter im Detail erläutert.

Fig. 1     zeigt den Sensor gemäß einem ersten bevorzugten Ausführungsbeispiel der Erfindung;

Fig. 2     zeigt einen Sensorelementaufsatz gemäß dem ersten bevorzugten Ausführungsbeispiel der Erfindung;

Fig. 3     zeigt einen Querschnitt durch den äußeren Gehörgang mit Sensorelement auf einfachem Sensorelementaufsatz gemäß einem zweiten bevorzugten Ausführungsbeispiel der Erfindung;

Fig. 4     zeigt einen Querschnitt durch den äußeren Gehörgang mit Sensorelement auf Doppelsensorelementaufsatz gemäß einem dritten bevorzugten Ausführungsbeispiel der Erfindung;

Fig. 5     zeigt eine Darstellung von einem Stabilisierungselement mit einer Feder, welche durch Druck auf die Ohrmuschel eine stabile Positionierung erzeugt gemäß einem vierten bevorzugten Ausführungsbeispiel der Erfindung;

Fig. 6     zeigt eine Darstellung eines weiteren Stabilisierungselement, welches in der Innenseite der Ohrmuschel positioniert werden kann und zum Halt des Sensors beiträgt gemäß einem fünften bevorzugten Ausführungsbeispiel der Erfindung;

Fig. 7     zeigt eine Prinzipdarstellung der Datenübertragung des Sensors an ein Mobilfunkgerät oder einen Computer gemäß einem sechsten bevorzugten Ausführungsbeispiel der Erfindung;

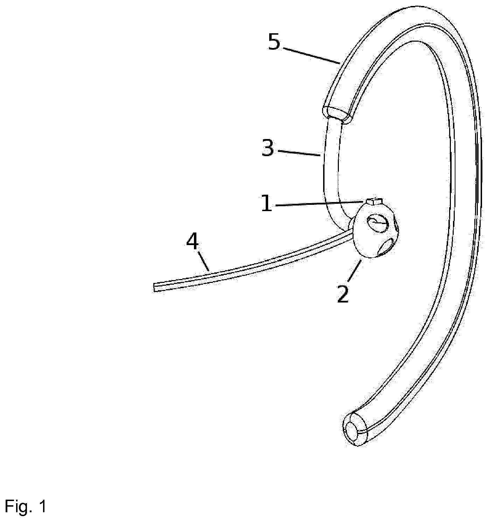

Fig. 1 zeigt einen Sensor gemäß einem ersten bevorzugten Ausführungsbeispiel der Erfindung. Dabei ist aus Fig. 1 zu erkennen, dass eine Auswerteeinheit 5 hinter dem Ohr über das anatomisch angepasste, vorgeformte Positionierungselement 3 mit dem Sensorelementaufsatz 2 verbunden wird. Zum zusätzlichen Halt des Sensors in der Ohrmuschel dient ein Stabilisierungselement 4. Das Sensorelement 1, vorliegend ein Temperatursensor, wird auf bzw. in dem Sensorelementaufsatz 2 befestigt. In der Auswerteeinheit 5 befinden sich eine Energiezelle, eine Auswerteelektronik sowie ein Sender.

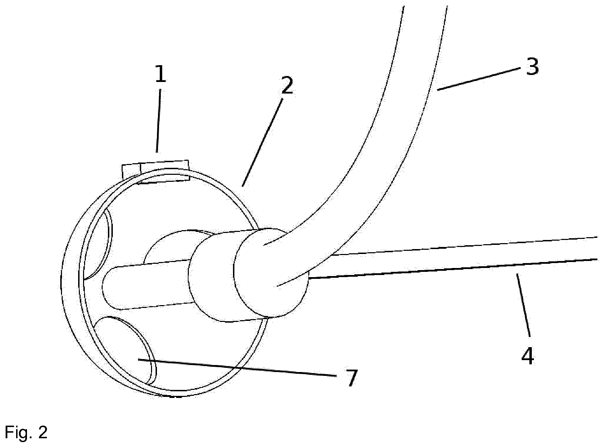

Fig. 2 zeigt den Sensorelementaufsatz 2 aus Fig. 1 gemäß dem ersten bevorzugten Ausführungsbeispiel der Erfindung. Der Sensorelementaufsatz 2 weist Aussparungen auf, um die Schallausbreitung nicht zu behindern. Auf den Sensorelementaufsatz 2 ist das Sensorelement 1, vorliegend der Temperatursensor, befestigt. Sensorelementaufsatz 2 und Positionierungselement 3 sind miteinander mechanisch verbunden.

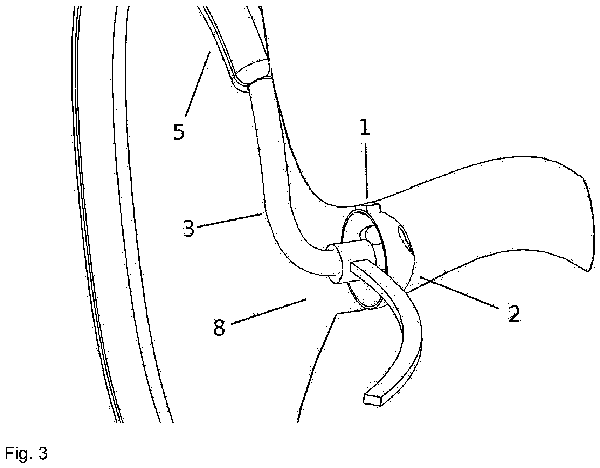

Fig. 3 zeigt einen Querschnitt durch den äußeren Gehörgang 8 und einen Teil des Sensors am Beispiel eines Temperatursensors gemäß einem zweiten bevorzugten Ausführungsbeispiel der Erfindung. Der Sensorelementaufsatz 2 ist mit dem Positionierungselement 3 mechanisch verbunden, wobei der Sensorelementaufsatz 2 das Sensorelement 1 trägt. Das Sensorelemente 1 kommen an der Haut zu liegen und befinden sich im äußeren Gehörgang 8 vor dem Trommelfell.

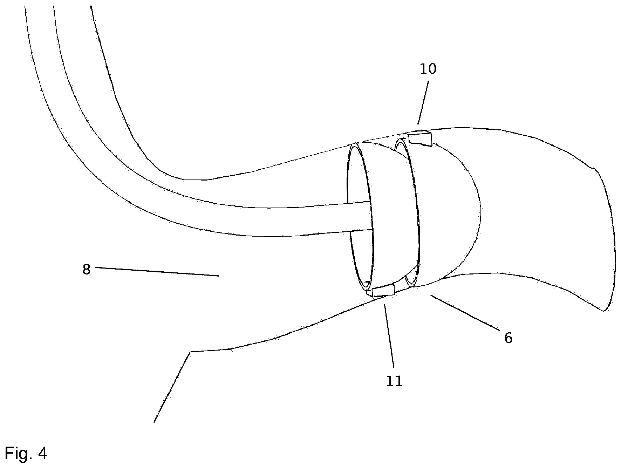

Fig. 4 zeigt einen Querschnitt durch den äußeren Gehörgang 8 und die im Gehörgang 8 befindlichen Komponenten Positionierungselement 3, Doppelsensorelementaufsatz 6, Lichtemitter 10 und Lichtempfänger 11 gemäß einem dritten bevorzugten Ausführungsbeispiel der Erfindung. Dies ist insbesondere für plethysmographische Sensorik, z.B. Pulsoximetrie im äußeren Gehörgang 8, geeignet. Es ist zu erkennen, dass eine optische Trennung der beiden Sensorelemente 10 und 11 durch den Doppelsensorelementaufsatz 6 zur Vermeidung von Shuntlicht erfolgt, d.h. der Lichtemitter 10 kann aufgrund der optischen Trennung durch den Doppelsensorelementaufsatz 6 den Lichtempfänger 11 nicht direkt, d.h. unter Umgebung eines Lichtwegs im Gewebe, erreichen. Außerdem erkennt man, dass der Lichtemitter 10 und der Lichtempfänger 11 in axialer Richtung gegeneinander versetzt an die Haut des Gehörgangs 8 gedrückt werden, wodurch eine Verlängerung des Lichtwegs und des Nettolichtwegs erreicht wird. Des Weiteren sind Lichtemitter 10 und Lichtempfänger 11 um etwa 180° in radialer Richtung gegeneinander versetzt.

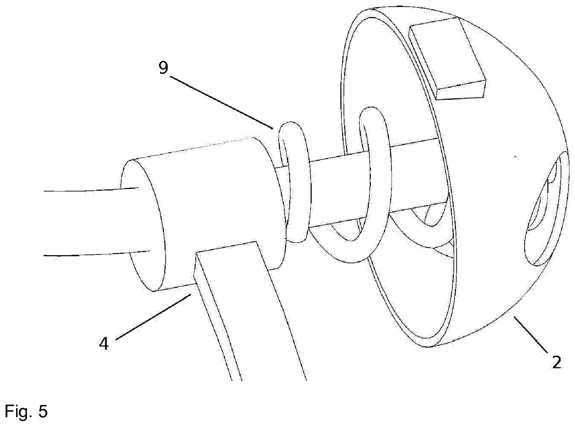

Fig. 5 zeigt ein Stabilisierungselement 4, welches durch eine Feder 9 nach außen gegen die Innenseite der Ohrmuschel gedrückt wird. Dadurch wird die Positionierung des Sensorelementaufsatzes 2 im Gehörgang erleichtert.

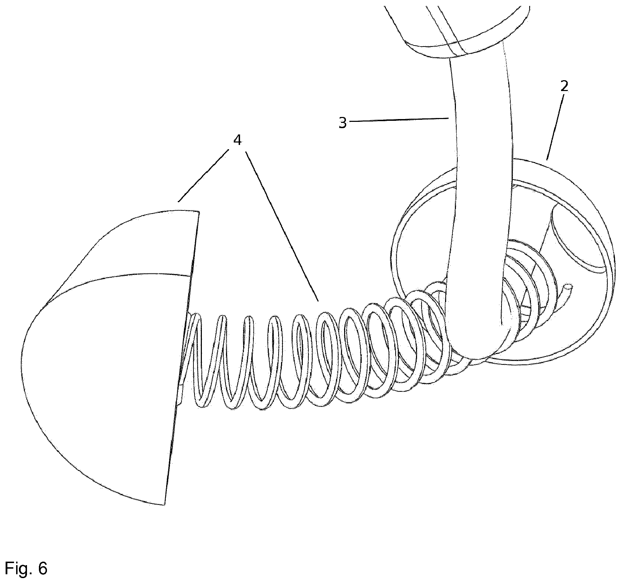

Fig. 6 zeigt ein Stabilisierungselement 4 gemäß einem weiteren Ausführungsbeispiel der Erfindung. Das Positionierungselement 3 verbunden mit dem Sensorelementaufsatz 2 werden durch das Stabilisierungselement 4 im Gehörgang stabilisiert und in Position gehalten.

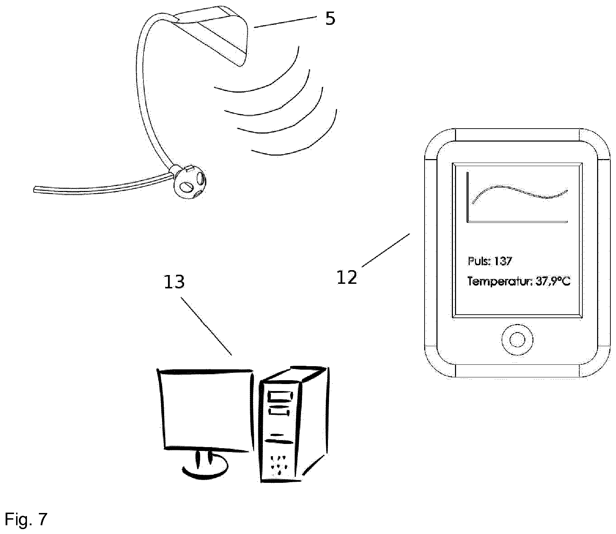

Fig. 7 zeigt den Aufbau des Sensors umfassend die in Fig. 1 detaillierter beschriebenen Komponenten gemäß einem sechsten bevorzugten Ausführungsbeispiel der Erfindung. Dabei sind drahtlos oder drahtgebunden angeschlossene Einheiten 12 und 13 in Form von Modulen zu erkennen. Die Auswerteeinheit 5 sitzt hinter dem Ohr. Außerdem erkennt man eine Anzeige- und Auswerteeinheit 13 und eine Mobilfunkeinheit 12, die räumlich getrennte Einheiten sein können, oder beliebig zusammengefasst werden können.

**Patentansprüche**

1. Sensor zur Messung eines Vitalparameters im Gehörgang (8) eines Menschen oder eines Tieres, umfassend:

   einen Sensorelementaufsatz (2), der wenigstens teilweise im Gehörgang (8) positionierbar ist,
   ein Sensorelement (1), das verbindbar mit dem Sensorelementaufsatz (2) ist und wenigstens teilweise im Gehörgang (8) positionierbar ist und einen Lichtemitter (10) und einen Lichtempfänger (11) umfasst, und ein Positionierungselement (3), geeignet zum Positionieren des Sensorelementaufsatzes (2) in den Gehörgang (8), wobei das Positionierungselement (3) an wenigstens einem Ende mit dem Sensorelementaufsatz (2) verbindbar ist, wobei die Position des Sensorelements (1) im Gehörgang (8) durch die Eindringtiefe des Sensorelementaufsatzes (2) in dem Gehörgang (8) bestimmt wird, wobei das Positionierungselement (3) eine geeignete, die Anatomie der Ohrmuschelinnenseite nachvollziehende Formgebung aufweist, und zum Drücken des Sensorelementaufsatzes (2) in das Innere des Gehörgangs (8) geeignet ist, derart, dass der Sensor stabil gehalten wird und eine kontinuierliche Messung des Vitalparameters im Gehörgang (8) erfolgt, wobei der Sensorelementaufsatz (2) mit geeigneten Löchern und/oder Aussparungen und/oder Kanälen vorgesehen ist, die gewährleisten, dass der Gehörgang (8) nicht vollständig verschlossen oder gedämpft wird, wobei der Sensorelementaufsatz als Material Silikon umfasst, **gekennzeichnet durch** eine Kontrolleinheit zur Überprüfung der Positionierung des Sensors im Gehörgang (8) und eine Anzeigeeinheit, wobei anhand der zeitlichen Änderung des Messsignals bei kontinuierlicher Messung des Vitalparameters die Position des Sensorelements (1) und/oder des Sensorelementaufsatzes (2) bestimmt wird und nach einer Änderung des Messsignals um einen vorbestimmten Wert die Abweichung der aktuellen Position von der gewünschten Position über die Anzeigeeinheit signalisiert wird und wobei die optimale Positionierung des Sensors über die Anzeigeeinheit signalisiert werden kann.

2. Sensor nach Anspruch 1, umfassend ein Stabilisierungselement (4), das zum Halt des Sensors in der Ohrmuschel geeignet ist und zumindest teilweise an dem Positionierungselement (3) und/oder an dem Sensorelementaufsatz (2) befestigbar ist.

3. Sensor nach Anspruch 2, wobei das Stabilisierungselement (4) wenigstens teilweise mit einer Feder (9) verbindbar ist, wobei die Feder (9) dazu geeignet ist, das Stabilisierungselement (4) gegen die Ohrmuschel zu drücken.

4. Sensor nach einem der vorhergehenden Ansprüche, wobei der Sensorelementaufsatz (2) derart elastisch ausgestaltet ist, dass er über seine Federkraft einen definierten Andruck auf die Wand des Gehörgangs (8) ausübt, so dass sich seine Form der Form des Gehörgangs (8) anpasst.

5. Sensor nach einem der vorhergehenden Ansprüche, wobei die Auswerteeinheit (5) an einem von dem Sensorelement (1) abgewandten Ende des Positionierungselements (3) an der Ohrmuschel positionierbar ist und geeignet ist, den Sensor stabil zu halten.

6. Sensor nach einem der vorhergehenden Ansprüche, umfassend einen Lautsprecher, der geeignet ist, ein Biofeedback über den gemessenen Vitalparameter im Gehörgang (8) wieder an das Innere des Gehörgangs (8) zurückzugeben.

7. Sensor gemäß Anspruch 6, wobei dieser die Einspeisung von akustischen Wellen, insbesondere im Bereich zwischen 0 und 20 kHz, ermöglicht.

8. Sensor nach einem der vorhergehenden Ansprüche, wobei der Sensorelementaufsatz gewölbt und/oder schirmförmig ausgestaltet ist.

9. Verfahren zur Bestimmung gewebsoptischer Größen, vorzugsweise der Sauerstoffsättigung im Blut, zur Durchführung von Pulsoximetrie, vorzugsweise Transmissions-Pulsoximetrie, im Gehörgang (8) unter Verwendung des Sensors gemäß einem der Ansprüche 1 bis - 8, umfassend den Schritt der Optimierung der Hilfsgröße Ω zur Bestimmung der Sauerstoffsättigung, vorzugsweise auf einen Wert von ≤ 0,5, besonders vorzugsweise auf einen Wert ≥ 0,4 und ≤ 0,5, ganz besonders vorzugsweise auf einen Wert von ungefähr 0,45, für humanes Oxihämoglobin und/oder für eine Wellenlänge, vorzugsweise von 740 nm und/oder 880 nm, oder für symmetrische Spektren von lichtemittierenden Dioden gleicher Zentralwellenlängen.

10. Verfahren gemäß Anspruch 9, wobei mindestens zwei Wellenlängen zwischen 660 nm und 1000 nm gewählt werden, besonders vorzugsweise Wellenlängen um 740 nm und/oder um 880 nm, ganz besonders vorzugsweise eine Wellenlänge ≤ 810 nm und eine Wellenlänge> 810 nm.

11. Verfahren gemäß einem der Ansprüche 9 oder10, wobei die Pulsfrequenz und/oder die Atemfrequenz bestimmt wird.

12. Verfahren gemäß einem der Ansprüche 9 bis 11, wobei die arterielle Sauerstoffsättigung und/oder die arteriell-venöse Sättigungsdifferenz ermittelt wird.

## Claims

1. Sensor for measuring a vital parameter in the ear canal (8) of a human person or animal, comprising:

   a sensor element fixture (2), which can be positioned at least partially in the ear canal (8), a sensor element (1), which can be connected to the sensor element fixture (2) and can be positioned at least partially in the ear canal (8) and which comprises a light emitter (10) and a light receiver (11), and a positioning element (3), suitable to position the sensor element fixture (2) in the ear canal (8), whereby the positioning element (3) can be connected to the sensor element fixture (2) on at least one end, wherein the position of the sensor element (1) in the ear canal (8) is determined by the insertion depth of the sensor element fixture (2) into the ear canal (8), wherein the positioning element (3) exhibits a suitable form, corresponding to the anatomy of the interior face of the auricle, and is suitable to press the sensor element fixture (2) into the interior of the ear canal (8) in such a manner, that the sensor is held stable and a continuous measurement of the vital parameter in the ear canal (8) is achieved, wherein the sensor element fixture (2) is provided with suitable holes and/or cavities and/or channels that ensure that the ear canal (8) is not completely closed or dampened, wherein the sensor element fixture comprises silicone as material, **characterized by** a control unit to monitor the positioning of the sensor in the ear canal (8) and a display unit, wherein the position of the sensor element (1) and/or of the sensor element fixture (2) will be determined on the basis of the temporal change of the measurement signal during continuous measurement of the vital parameter, and after a change in the measurement signal by a predetermined value, the difference between the current position from the desired position will be indicated via the display unit and wherein the optimum position of the sensor can be indicated by a display unit.

2. Sensor according to claim 1, comprising a stabilizing element (4), which is suitable to retain the sensor in the auricle, and can be attached at least partially to the positioning element (3) and/or to the sensor element fixture (2).

3. Sensor according to claim 2, wherein the stabilizing element (4) can be connected at least partially to a spring (9), wherein the spring (9) is suitable to press the stabilizing element (4) against the auricle.

4. Sensor according to any of the preceding claims, wherein the sensor element fixture (2) is formed elastically in such a manner, that through its spring force, it exerts a defined pressure on the wall of the ear canal (8), so that it adapts its form to the form of the ear canal (8).

5. Sensor according to any of the preceding claims, wherein the evaluation unit (5) can be positioned on the auricle on an end of the positioning element (3) opposite to the sensor element (1) and is suitable to hold the sensor stable.

6. Sensor according to any of the preceding claims, comprising a speaker that is suitable to return biofeedback about the vital parameters measured in the ear canal (8) back to the interior of the ear canal (8).

7. Sensor according to claim 6, wherein the sensor enables the introduction of acoustic waves, in particular in the range between 0 and 20 kHz.

8. Sensor according to any of the preceding claims, wherein the sensor element fixture is curved and/or formed as a screen.

9. Process for determining optical tissue parameters, preferably the oxygen saturation in the blood, for performing pulse oximetry, preferably transmission pulse oximetry, in the ear canal (8) using the sensor according to any one of the claims 1 to 8, comprising the step of optimizing the auxiliary dimension $\Omega$ for the determination of oxygen saturation, preferably to a value of $\leq 0.5$, particularly preferably to a value $\geq 0.4$ and $\leq 0.5$, especially particularly preferably to a value of approximately 0.45, for human oxihemoglobin and/or for a wavelength, preferably of 740 nm and/or 880 nm, or for symmetric spectra of light-emitting diodes of the same central wavelength.

10. Process according to claim 9, wherein at least two wavelengths are selected between 660 nm and 1000 nm, particularly preferably wavelengths around 740 nm and/or around 880 nm, especially particularly preferably one wavelength $\leq 810$ nm and one wavelength > 810 nm.

11. Process according to claim 9 or 10, wherein the pulse rate and/or the respiration rate are determined.

12. Process according to any of claims 9 to 11, wherein the arterial oxygen saturation and/or the arterial-venous saturation difference are determined.

**Revendications**

1. Capteur pour la mesure d'un paramètre vital dans le conduit auditif (8) d'un être humain ou d'un animal, comprenant :

un embout d'élément de capteur (2), pouvant au moins en partie être mis en place dans le conduit auditif (8),
un élément de capteur (1), pouvant être raccordé à l'embout d'élément de capteur (2) et pouvant au moins en partie être mis en place dans le conduit auditif (8), et comprenant un émetteur de lumière (10) et un récepteur de lumière (11), et un élément de positionnement (3) prévu pour mettre en place l'embout d'élément de capteur (2) dans le conduit auditif (8), où l'élément de positionnement (3) peut être raccordé à l'embout d'élément de capteur (2) à au moins une extrémité, où le positionnement de l'élément de capteur (1) dans le conduit auditif (8) est déterminé par la profondeur de pénétration de l'embout d'élément de capteur (2) dans le conduit auditif (8), où l'élément de positionnement (3) présente une forme adaptée, conforme à l'anatomie de l'intérieur du pavillon auriculaire, et permet l'insertion de l'embout d'élément de capteur (2) à l'intérieur du conduit auditif (8), de manière à obtenir un maintien stable du capteur et une mesure continue du paramètre vital dans le conduit auditif (8), l'embout d'élément de capteur (2) étant pourvu de trous et/ou d'évidements et/ou de canaux adaptés, lesquels assurent que le conduit auditif (8) n'est pas complètement obturé ou bouché, l'embout d'élément de capteur comprenant du silicone en tant que matériau,

**caractérisé par**

une unité de contrôle pour la surveillance du positionnement du capteur dans le conduit auditif (8) et une unité d'indication, la position de l'élément de capteur (1) et/ou de l'embout d'élément de capteur (2) étant déterminée sur la base de la variation dans le temps du signal de mesure lors de la mesure continue du paramètre vital, et l'écart entre la position actuelle et la position souhaitée étant signalé par l'unité d'indication après une variation d'une valeur définie du signal de mesure, et le positionnement optimal du capteur pouvant être signalé par l'unité d'indication.

2. Capteur selon la revendication 1, comprenant un élément de stabilisation (4), prévu pour maintenir le capteur dans le pavillon auriculaire et pouvant être au moins partiellement fixé contre l'élément de positionnement (3) et/ou contre l'embout d'élément de capteur (2).

3. Capteur selon la revendication 2, où l'élément de stabilisation (4) peut être au moins partiellement raccordé à un ressort (9), ledit ressort (9) étant prévu pour contraindre l'élément de stabilisation (4) contre le pavillon auriculaire.

4. Capteur selon l'une des revendications précédentes, où l'embout d'élément de capteur (2) est conçu avec une élasticité lui permettant d'exercer par sa force de ressort une pression définie sur la paroi du conduit auditif (8), de manière à ajuster sa forme à la forme du conduit auditif (8).

5. Capteur selon l'une des revendications précédentes, où l'unité d'évaluation (5) est positionnable contre le pavillon auriculaire à une extrémité de l'élément de positionnement (3) distante de l'élément de capteur (1), et est prévue pour un maintien stable du capteur.

6. Capteur selon l'une des revendications précédentes, comprenant un haut-parleur prévu pour retourner vers l'intérieur du conduit auditif (8) un biofeedback relatif au paramètre vital mesuré dans le conduit auditif (8).

7. Capteur selon la revendication 6, où celui-ci permet l'injection d'ondes acoustiques, en particulier dans la plage entre 0 et 20 kHz.

8. Capteur selon l'une des revendications précédentes, où l'embout d'élément de capteur est voûté et/ou en forme de parapluie.

9. Procédé de détermination de grandeurs optiques tissulaires, préférentiellement de la saturation en oxygène du sang, pour effectuer une pulsoxymétrie, préférentiellement une pulsoxymétrie par transmission, dans le conduit auditif (8) au moyen du capteur selon l'une des revendications 1 à 8, comprenant l'étape d'optimisation de la grandeur auxiliaire $\Omega$ pour déterminer la saturation en oxygène, avantageusement à une valeur $\leq 0,5$, préférentiellement à une valeur $\geq 0,4$ et $\leq 0,5$, tout particulièrement à une valeur sensiblement égale à 0,45, pour l'oxyhémoglobine humaine et/ou pour une longueur d'onde, préférentiellement de 740 nm et/ou de 880 nm, ou pour des spectres symétriques de diodes électroluminescentes de mêmes longueurs d'ondes centrales.

10. Procédé selon la revendication 9, où au moins deux longueurs d'onde entre 660 nm et 1000 nm sont sélectionnées, préférentiellement des longueurs d'onde de 740 nm et/ou de 880 nm, tout particulièrement une longueur d'onde $\leq 810$ nm et une longueur d'onde > 810 nm.

11. Procédé selon la revendication 9 ou la revendication 10, où la fréquence cardiaque et/ou la fréquence respiratoire sont déterminées.

12. Procédé selon l'une des revendications 9 à 11, où la saturation artérielle en oxygène et/ou la différence de saturation artério-veineuse sont déterminées.

Fig. 1

Fig. 2

Fig. 3

Fig. 4

Fig. 5

Fig. 6

Puls: 137

Temperatur: 37,9°C

5

12

13

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1922989 A
- DE 102007046295